# EUROPEAN PATENT SPECIFICATION

(11) **EP 4 212 096 B1**
(45) Date of publication and mention of the grant of the patent: **09.09.2026**
(21) Application number: 22151488.8
(22) Date of filing: 14.01.2022
(51) Int. Cl.: A61B 5/20, A61B 10/00, A61B 5/145, A61B 5/1455

(54) **DEVICE FOR MEASUREMENT OF URINE FLOW AND A SYSTEM FOR DIAGNOSTICS OF MICTURITION DISORDERS COMPRISING SAID DEVICE**
VORRICHTUNG ZUR MESSUNG DES HARNFLUSSES UND SYSTEM ZUR DIAGNOSE VON MIKTIONSSTÖRUNGEN MIT DIESER VORRICHTUNG
DISPOSITIF DE MESURE DE DÉBIT URINAIRE ET SYSTÈME DE DIAGNOSTIC DE TROUBLES DE LA MICTION COMPRENANT LEDIT DISPOSITIF

(43) Date of publication of application: 19.07.2023
(73) Proprietor: Dedisol d.o.o., 2000 Maribor (SI)
(72) Inventor: STOZER, Andraz, 2000 Maribor (SI); KRAMBERGER, Iztok, 2000 Maribor (SI); PROSEN, Sergej, 2000 Maribor (SI); NIKOLIC, Gregor, 2000 Maribor (SI)
(74) Representative: Patentni Biro AF d.o.o.

(56) References cited:
- EP-A2- 0 152 644
- WO-A1-2014/141234
- KR-A- 20130 095 869
- US-A1- 2015 342 574
- US-A1- 2017 105 670

## Description

### Field of the invention

The invention belongs to the field of medicine, more precisely to the field of diagnostic devices, particularly devices for measurement of urine flow. The present invention relates to a device for measurement of urine flow and a system for diagnostics of micturition disorders comprising the said device.

### Background of the invention and the technical problem

Urine is a fluid excreted by kidneys through the urinary tract. Urine flows from the kidneys through the ureters to the urinary bladder and then leaves the body through the urethra. Urinary excretion is called urination or micturition. The formation of urine regulates the amount and composition of body fluids and allows excretion of metabolites from the body. Many diseases are reflected in the appearance, quantity and composition of urine; hence, urine analysis is an important diagnostic method. Micturition disorders are common problems, as every second woman and every third man experience them at a given time. Clinical conditions that can cause urinary disorders include incontinence, overactive bladder, inability to urinate, inflammation, prostatitis, prostate enlargement, polyuria, oliguria, anuria, bladder and urethral sphincter incoordination as a result of brain and/or spinal cord injury. Inflammation of the bladder and involuntary leakage of urine are common in women, while in men most problems are due to the gonads of the prostate and overactive bladder. Disorders can be an indicator or a prediction of serious diseases, such as prostate cancer, which makes monitoring the condition necessary in order to ensure timely action and treatment.

Given the prevalence of urinary disorders in the general population and the projected aging of the population, the need for accurate and accessible diagnostics is high and will continue to increase in the future. Measurements of urine flow are classified as non-invasive tests, but they can nevertheless cause a feeling of discomfort and shame in patients, and even more often in women patients. A 2019 study found that anxiety due to urine flow measurements occurred in 40% of participants and significantly affected patients 'perceptions of the reproducibility of the test compared to their normal urination patterns (Rubilotta, 2019; doi: 10.1186/ s12894-019-0468-2). It is also known that normal physiology requires proper physiological conditions and that artificially created conditions and lack of privacy can significantly affect urine flow (Ather, 1998; Tech Urol 4 (3): 111-7). Consequently, it is recommended that the assessment of urine flow is performed in a familiar, home environment. In addition, a representative result of flow measurements of urine requires a full bladder, which is not always feasible during the examination in a clinic or hospital due to waiting time. In addition, it is also known that the results of urine flow measurements can vary during the day, hence more measurements are needed for a precise diagnosis of micturition problems (de la Rossette, 1996, British Journal of Urology, 78: 358-390).

Up to now several different devices for measurement urine flow for home use have been developed, however, the majority of these devices is suitable for use in men population. Thus, the population of women has been overlooked even tough micturition disorders are more common in women than in men.

Furthermore, some accessories for semiquantitative or quantitative measurement of maximal urine flow (Qₘₐₓ) are also known, but this is only one of the important parameters of urine flow. Among those, Smith device, PEL device, UFlow Meter and Peakometer can be mentioned, wherein their biggest limitation is the fact that they enable measurement of a single parameter. Therefore, these devices are not suitable for comprehensive and in-depth treatment of patients.

Despite the fact that several devices are present in the market that allow measurement of urine flow at home, their wider use is limited by the relatively high price and their suitability for use only in men. With development of an affordable urine flow measurement device for home use that is also suitable for women, numerous possible uses in uro-gynaecology and urologic clinics would be created.

Therefore, the technical problem, which is solved by the present invention, is construction of a device for measurement of urine flow and a system for diagnostics of micturition disorders comprising the said device that will allow:
- reliable measurements in women and men at home and in clinical environments,
- basic diagnostics of micturition disorders without a specialized device for urodynamic examination, which are currently only performed in tertiary centres,
- objective recording of a urination diary, which is extremely important for the definition of micturition disorders and decisions about further treatment,
- keeping track of effects of therapy, such as behavioural measures, pharmacological therapy, continence operations, botulinum toxin application into the bladder, and
- objective detection of possible deterioration.

Additionally, the aim of the invention is to ensure that the device is affordable, which represents an important aspect in design of diagnostic devices.

### Prior art

Until now the following devices for measurement of urine flow for home use have been described in the literature or on the internet.

Home-based uroflowmeter (de la Rossette et al., 1996, British Journal of Urology, 78: 358-390) comprises a bowl-shaped volume sensor for single use. Home UroData System produced by company Biodan Medical Systems was designed as a collection device in the shape of a truncated cone and enabled tracking of frequency and volume of several consecutive mictions.

Wireless Uroflowmetry System designed by company Bestmedical is a transportable system for urine flow measurements having a collection device shaped as a funnel. It allows measurement of urine flow and the total urine volume, wherein the device is based on a gravimetric method or a scale, respectively. The device comprises batteries, while data transfer is done via a Bluetooth module. Men can give samples directly into the device installed on a holder, while women need a dedicated chair.

MenHealth Mobile UroFlowmetry produced by the company BE Technologies is a system developed for men. It enables measurement of highest urine flow, total urine volume and micturition time as well as keeping a diary via a mobile application. The parameters are calculated on the basis of detected sound of urine contacting the water in a toiled bowl. The sound-based measurement corelates with urodynamic measurements, but does not allow precise quantification.

PeePod of the company Tyne Hospitals, NHS Foundation Trust, is a urine flow measurement device for single use, which was also developed for use in men population. It allows measuring urine flow and keeping a diary of urination in time intervals up to two months. It is not suitable for use in women population.

iUFlow produced by Kesem Health comprises a urine flow measuring device for home use, which is mounted on a toilet bowl and is then connected to a mobile application installed on a smart phone in order to track the results. Patent US10130293B2 related to this device describes a bowl-shaped collecting device and is technologically based on collection of acoustic signals. The lifespan of the device is 4 weeks or 60 measurements.

All described devices have in common that the measuring device has a round shape like a bowl or a funnel, which significantly differs from the present invention.

Document US9974520 relates to a device and a method for automatic collection of urine samples, wherein the device comprises a toilet bowl with a container for urine collections, said container being a flexible plastic bag, which can be thermally welded. This solution differs from the present invention, which does not use plastic bags.

Patent application JPH0257240 discloses a device for controlling urine collection with a purpose of simple measurement of urination time and calculation of a mean flow volume by measuring a flow volume by so that a potential difference between two points of capacitance bridges is connected to a changing capacitance value. The device comprises electrodes and a microprocessor, but functions in a different manner as the present invention, wherein also the shape of the device is different.

Patent application JPH11326316 discloses a toilet device with a function for urine analysis, wherein the urine analysing unit is formed of a housing of the device, a urine transferring pump and a stool seat assembly, in which a urine collection device is incorporated. The latter has electrodes for sensing the amount of urine. Transfer and analysis of urine is performed only if a predetermined amount of urine is present. This solution has separate units for collection and analysis of urine, which complicates the design and operation of the system.

Document US5062304 describes an apparatus and a method for flow and temperature analysis during collection of a urine sample, which at the same time maintains the urine sample sterile. The device for collecting urine comprises a variable capacitor, which forms a part of a capacitance bridge. A microprocessor controlled by a clock is used to periodically sample the varying capacitance value as fluid flows into the device, and thereby determines the flow rates of the fluid. A separate tube is provided which communicates with the interior of the device for drawing off a sterile sample of the urine for laboratory analysis, while temperature sensing means are provided for detecting the temperature of the fluid as a function of time.

Patent application WO2014141234 relates to a system for measuring a volume and flow rate of urine, which comprises : (A) a vessel which comprises : (a) a first wall; (b) a bottom capacitor and plurality of ring capacitors, each of said capacitors comprises an external plate, a dielectric material and an internal plate, wherein the area of said internal plate is determined by the volume of urine within the vessel, and by a possible slant orientation of the vessel; (c) a common electrode within the vessel, which is in contact with urine! (B) a data collection circuit, which comprises : (d) a capacitance depending oscillator: (f) a multiplexer for connecting one at a time one of said capacitors to said oscillator; and (g) a processor for controlling said multiplexer to sequentially select one at a time another capacitor from said capacitors, for determining a respective oscillator frequency resulting from each of said selections, and for storing within a memory for each such selection a respective frequency and time stamp, all said storage forms a raw data; and said memory for storing said raw data. The shape of the receptacle for urine is a cone, preferably a truncated cone. This document also cites other shapes can be used, but does not proceed to describe any other shapes. Hence, the only disclosure is the cone-shaped receptacle.

Patent application US2017105670 discloses a urine measurement device includes: a container portion configured to receive a substance; a sensing device configured to measure a property of the substance related to at least one of a flow rate of the substance into the container portion, a level of the substance within the container portion, and a volume of the substance within the container portion; and at least one shield positioned within the container portion and configured to provide at least one of: a mechanical buffer between the substance entering the container portion and the sensing device; and an electrical shield between the substance entering the container portion and the sensing device. The urine receiving container is shaped as a tapered cone, which is similar to the shape used in the above-mentioned WO2014141234.

### Description of the solution to the technical problem

The technical problem is solved as defined in the claims.

The device for measurement of urine flow according to the invention comprises at least two containers, an inner container and an outer container, wherein the device is made from a non-rigid resilient material and comprises:
- at least two containers, an inner container and an outer container, wherein the inner and the outer container are shaped as a truncated pyramid or truncated prism with six sides, three narrower and three wider sides arranged in an alternating manner, and the inner container is arranged to be installed inside the outer container, wherein the inner and the outer container may be separate pieces or may be joined into one piece having the said shape,
- the outer container has an elongated neck and a bottom for receiving the inner container, wherein the inner container ends with an inner bottom arranged to collect urine, wherein the said inner bottom is from the outer surface provided with electronics for ensuring operation of the device, thus said electronics being protected between both bottoms,
- flex capacitive electrodes glued on the wider sides of the truncated prism or pyramid, on the outer surface of the inner container, said flex capacitive electrodes provided with a shield, arranged to detect the level of urine on each of the wider sides, wherein the flex capacitive electrodes are covered by the outer container, thus protecting the flex capacitive electrodes from environment, preferably the edges of the inner and the outer container are sealed in any suitable manner that allows sealing, moisture, water and/or dust protection, as well as reliable operation of the electrodes, and wherein three electrodes ensure precise measurements due to averaging level of fluid using triangulation method and minimizing the effect of waves and/or inclinations due to non-horizontal installation of the toilet bowl,
- the bottom of the device is also provided with at least three condensers and capacitive bridges formed by the flex capacitive electrodes and pins of capacitance measurement devices connected to a printed circuit,
- at least one sensor for sensing movement and tilting of the device, preferably an accelerometer and a gyroscope, and
- preferably at least one temperature sensor arranged to detect urine temperature, wherein the said temperature sensor protrudes from the bottom into the interior of the device.

Due to urine collection in the bottom of the inner container the sample may be used for any other analysis, such as standard biochemical urine analysis.

Advantages of the pyramid-shaped container are:
- due to straight sides flat electrodes are installed in a simple and stable manner,
- due to the shape of the container, which is narrowest on the top of the pyramid (bottom of the container), the changes in the height of the urine surface are highest for a given volume at the beginning of urination and are then reduced, which ensures good resolution at the beginning of urination.

As the container is narrower at the bottom, higher sensitivity of initial measurement is ensured, as the container is filling up faster and waves as well as spraying are reduced. In this part of micturition (urination) the maximal flow is achieved, hence, it is important to precisely cover this part of the measurement in order to precisely determined the maximal flow and time needed to achieve maximal flow.

The number of sides of the pyramid-shaped containers may be higher than three. However, according to the invention, the container has six sides, wherein three are narrower and three are wider and are present in an alternating manner (narrower-wider-narrower... ), and the electrodes are installed on the wider sides. At least three sides, each with at least one electrode are the minimum solution for sensing possible tilt of the fluid surface. Orientation of the sides is such that upon installation of the device on the toilet bowl the urine does not flow directly on electrodes. Preferably, the inner and the outer container are separate pieces, in order to provide a space for installation of electrodes and at the bottom a space for installation of electronics, which is very useful. The connection between both parts can be sealed or otherwise glued. The device may be made from any suitable resilient material that is washable, particularly from thermoplastics. The selected material thus ensures a long life-span of the container and improved hygienic properties.

Additionally, the device may be provided with a separate holder, which is arranged to hold the above-described device above the toilet bowl. The holder may have any suitable shape, which allows reliable installation on any toilet bowl. The holder is not essential, as the above-described device can be placed on the floor or on a chair, which does not affect the measurement and its validity. If present, the holder is preferably shaped as a flat element with a central part with an opening in the middle, said opening being arranged to receive the device, and wherein the central part of the holder has at least two sides (or wings) for installation on the toilet bowl. The holder may also be replaced separately from the device and by adapting its shape firm placement on the toilet bowl is ensured. Said holder may be made from stiffer material than the device and may be plastics or any other suitable material.

The sensor for sensing movements and/or tilting of the container and is preferably IMU 6 axial sensor (accelerometer and gyroscope) and is installed on the printed circuit of the device, which is provided in the double bottom of the device. The accelerometer may optionally have a function for controlling operation of the device, ON/OFF, wake up/sleep with regards to the settings defined by the user. The gyroscope is arranged to sense any possible movements and tilting of the device, so that in case of non-optimal position of the device a suitable correction in the electrodes may be performed, thus ensuring reliable data in spite of non-optimal measurement position.

The flex capacitive electrodes are used in the device, said electrodes being glued to the outer surface of the inner container. The device is made of non-rigid material, to which the flex electrode adapts better. The elasticity of the housing and the electrode eliminates possible movements of the device and prevents possible mechanical separation of the electrode from the inner container of the device. The electrodes are connected to the printed circuit board in known ways, which is obvious to a person skilled in the art.

Additionally, the device may be provided with a sound sensor and/or a spectrophotometric device. The sound (acoustic) sensor or a microphone, respectively, is arranged to detect urine dripping, which may indicate urinary retention and premature closure of the urethra or diminished detrusor muscle, which may be an important diagnostic parameter. In addition, the microphone may further contribute to precise detection of micturition initiation, even before the fluid reaches the electrodes. The device may be equipped with a suitable spectrophotometric device, preferably a 12-channel sensor for spectrophotometry, wherein the collected urine is illuminated at different wavelengths, thereby measuring and estimating presence of particular substances in urine. This may eliminate the need for chemical analysis of the collected urine or the spectrophotometric analysis with the device may be confirmed with a further chemical analysis. In this manner presence of blood, protein, glucose or particles in urine may be detected, which indicates an inflammation, presence of bacteria and/or renal sand. The spectrophotometric device may sense first droplets of urine in the container, even before the fluid reaches the electrodes, which further contributes to precise detection of micturition initiation and analysis of the initial phase of urination.

The device may be further equipped with additional sensors and indicators in the bottom of the container, such as for example indicator lights for feedback about device operation. Preferably, the device is equipped with an LED RGB light (green, blue, red) integrated on the printed circuit board. Said LED can be arranged to create diffuse reflection, especially if the device is milky white. For example, when the user lifts the container, the accelerometer detects movement and turns on the device, the device can then reflect blue. A ready-to-measure device may flash blue, when it starts measuring liquid, it may glow green, in case of an empty battery it may flash red, etc. Additionally or alternatively, the device may also have a display that can show the numbers at the bottom of the device. After the measurement, when the user turns the device over in order to empty it, the display at the bottom shows the values of the measured parameters, which the user may write down.

The device preferably has additional electronic components, which allow for autonomic operation of the device, namely:
- a memory for storing data from sensors and electrodes,
- a processor for processing said data,
- a suitable transmitter for transferring said data in any suitable way, preferably via Bluetooth,
- a power source and a suitable battery, which may be any battery, a rechargeable or a replaceable battery.

The software is installed with the aim to enable device operation and is arranged to perform the following steps:
- collecting data forwarded from the sensors,
- processing of received data, in order to calculate or read the information about maximal and average flow, urine volume and urination time,
- storing data for each sample or for each patient, respectively, wherein the data may be stored in order to gain a time sequence of measurements for individual patient, which allows precise monitoring of the state of micturition disorders (improvement, deterioration, stagnation).

Values of parameters are sent to the processor and memory of the device and are then suitable processed and sent in any suitable manner to a cloud or application, where they are stored and thus available to the patient as well as the physician. The device may be configured in such way to automatically transmit data into a cloud via 5G network.

A system for diagnostics of micturition disorders comprises the device for measurement of urine flow as described above, and an online analytical application or a platform and a mobile application installed on a mobile or smart phone, tablet or a similar user device. The online and mobile applications are intended to show measurement data and can be designed and programmed in any suitable manner known to the person skilled in programming.

During use of the device and the system as described above, information about maximal and average flow, volume and time of urination is obtained. Namely, the device gives an information about millilitres per second with a time resolution of at least one point per second, preferably 10 points per second. With analysis the following can be read or calculated:
- total time of urination,
- total volume,
- maximal urine flow, and
- time needed to reach the maximal urine flow.

These are standard uroflowmetry data given by invasive diagnostic devices in clinical centres and thus represent parameters and imaging which medical doctors (and patients) are familiar with.

The device and the system may be used in diagnostics of urinary disorders, in monitoring the status of different disorders and diseases, as well as in general urological or uro-gynecological examinations. The method of use of the device and the system is the following:
a) installation of the device on a suitable flat surface or preferably installation of the device in one piece (joined inner and outer container) on a toilet bowl via a holder with an opening arranged to receive the device,
b) collecting urine sample into the device prepared in step a), wherein electrodes measure the fluid level on each wall of the device where electrodes are installed, and wherein the measurements are averaged and optionally adjusted with regards to the sensed urine temperature, tilt of the device or similar,
c) preferably sensing sounds of urine drops and/or spectrophotometric analysis of collected urine,
d) preferably sensing tilt of the device or its movement with an accelerometer and/or gyroscope,
e) sending data obtained from electrodes and sensors in steps b), c) and/or d) in real time to a microprocessor and memory of the device,
f) processing of the data acquired in step e) in order to obtain at least one, preferably all of the following parameters:
   - total time of urination,
   - total volume,
   - maximal urine flow, and
   - time needed to reach the maximal urine flow.

Steps b), c) and d) may be carried out simultaneously, sequentially, two simultaneously and one later on or in any other sequence in any meaningful combination obvious to a skilled person. For example, movement of the device may be sensed during collection and sample analysis, while it can also be sensed simultaneously with sample collection. The spectrophotometric analysis and/or acoustic analysis may be performed even before sensing with capacitive electrodes, but it can alternatively or additionally perform at a later stage, when the capacitive electrodes perform or finish the measurement.

The device according to the invention has an important role in diagnostics of micturition disorders in women as well as in men, while one or more measured parameters are not sufficient for a reliable disorder diagnosis. Particular parameter values may suggest urethra obstruction (lower flow, usually under 15 or 12 ml/s), urgency urinary incontinence (increased flow, usually above 25 mL/s), however, these indications need further inspection. In combination with other methods, such as determination of remaining urine volume after micturition (post-void residual volume - PVR), the device according to the invention enables reliable monitoring of the patient and therefore simplified treatment, which is also the aim of the invention.

The present invention thus successfully solves the technical problem and enables:
- an affordable device that provides reliable measurements for women and men in home and clinical setting,
- basic diagnostics of micturition disorders without a specialized device for urodynamic examinations, so that basic diagnostics can also be performed in urogynecological and urological clinics at the secondary level,
- objective recording of the urination diary, which is extremely important for the definition of micturition disorders and decisions regarding further treatment,
- monitoring the success of therapeutic measures such as behavioural measures, pharmacological therapy, continence surgery, application of botulinum toxin to the bladder, and
- objective detection of possible deterioration.

The device for measurement of urine flow and the system for diagnostics of micturition disorders comprising the said device according to the invention will be described in further detail based on exemplary embodiments and figures, which show:
- Figure 1: A device for measurement of urine flow according to a possible embodiment
- Figure 2: The device for measurement of urine flow according to a possible embodiment shown in figure 1 with a shown position of electrodes and sensors, wherein the outer part is partly cut away in order to show the bottom and the interior of the device
- Figure 3: Exemplary results of the analysis with the system according to a possible embodiment

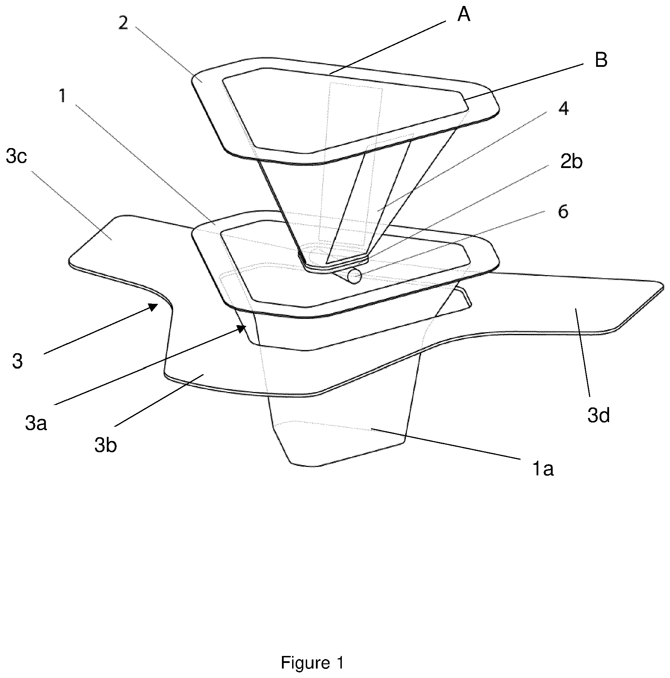

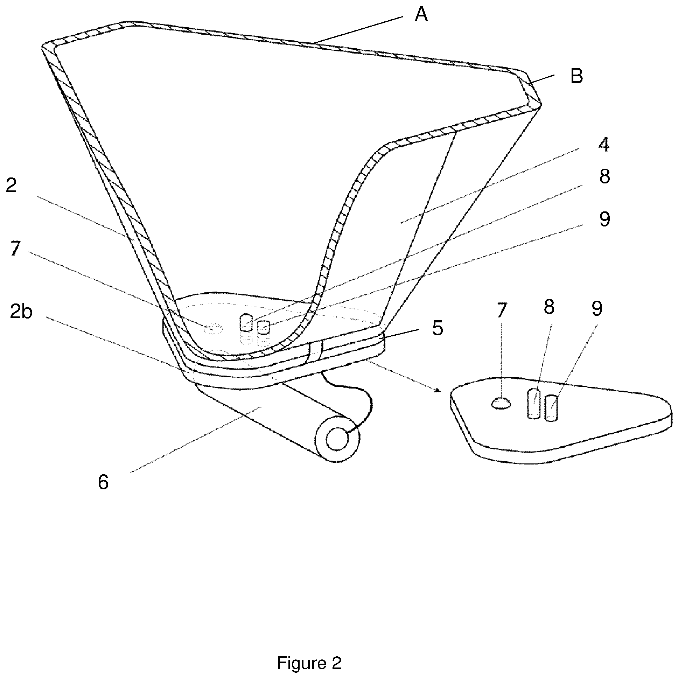

Figures 1 and 2 show the device for measurement of urine flow made of a non-rigid resilient material according to a possible embodiment, which comprises:
- an inner container 2 and an outer container 1, which are both shaped as a truncated pyramid with three wider sides A and three narrower sides B, wherein the inner container 2 is installed in the outer 1 and the said outer container 1 has an elongated neck 1a with a bottom, while the inner container 2 ends with an inner bottom 2b arranged to collect urine, wherein said inner bottom 2b is on its outer surface provided with electronics of the device, which ensures operation of the device, so that the electronics are protected in the double bottom of the device,
- the outer surface of each of the three wider sides A is provided with one flex capacitive electrode 4 arranged to sense the level of urine on each side of the device, wherein the flex capacitive electrodes are covered with the outer container in order to protect them against environmental conditions, and the edges of the inner and the outer containers are sealed, and
- at least three condensers and capacitive bridges formed by the flex capacitive electrodes and pins of capacitance measuring devices, and a central controller on a printed circuit, said components being installed in the space 5 between both bottoms,
- a battery 6 for ensuring energy for operation of the device and its components installed under the inner bottom 2b,
- at least of sensor for sensing movement and tilting of the device, preferably an accelerometer and a gyroscope,
- a spectrophotometric sensor 7, which protrudes through the bottom 1b into the interior of the device and is arranged to perform spectrophotometric analysis of the fluid collected in the device,
- a microphone 8 installed on the bottom 2b next to the spectrophotometric sensor, wherein the microphone is entirely under the bottom and does not protrude into the interior of the device, and
- a temperature sensor 9 for measuring temperature of the urine collected in the bottom 1b of the device, wherein the sensor protrudes into the interior of the device through the bottom 2b in the same manner as the spectrophotometric sensor, and wherein the said temperature sensor is arranged to measure fluid temperature,

The device is installed on the holder 3, which is shaped so that the above-described device is held above the toilet bowl. The holder has a flat central part with an opening 3a for receiving the device and three sides 3b, 3c, 3d for installation on the toilet bowl.

The system for diagnostics of micturition disorders according to a possible embodiment comprises the device for measurement of urine flow as shown in figures 1 and 2 and a smart device, such as a smart phone, a tablet computer or a computer, as well as a suitably programmed application or a program arranged to show results of measurements with the device to a user, which is a patient or a medical doctor (physician).

The device and the system may be used in diagnostics of urinary disorders, in monitoring the status of different disorders and diseases, as well as in general urological or uro-gynecological examinations. The method of use of the device and the system according to the described embodiment is the following:
a) installation of the device in one piece (i.e., the inner container 2 and the outer container 1 are joined) on a toilet bowl via the holder 3 with an opening arranged to receive the device,
b) collecting urine sample into the device prepared in step a), wherein electrodes measure the fluid level on each wall of the device where electrodes are installed, and wherein the measurements are averaged and optionally adjusted with regards to the sensed urine temperature, tilt of the device or similar,
c) preferably sensing sounds of urine drops and/or spectrophotometric analysis of collected urine,
d) preferably sensing tilt of the device or its movement with an accelerometer and/or gyroscope,
e) sending data obtained from electrodes and sensors in steps b), c) and/or d) in real time to a microprocessor and memory of the device,
f) processing of the data acquired in step e) in order to obtain at least one, preferably all of the following parameters:
   - total time of urination,
   - total volume,
   - maximal urine flow, and
   - time needed to reach the maximal urine flow.

Steps b), c) and d) may be carried out simultaneously, sequentially, two simultaneously and one later on or in any other sequence in any meaningful combination. For example, movement of the device may be sensed during collection and sample analysis, while it can also be sensed simultaneously with sample collection. The spectrophotometric analysis and/or acoustic analysis may be performed even before sensing with capacitive electrodes, but it can alternatively or additionally perform at a later stage, when the capacitive electrodes perform or finish the measurement.

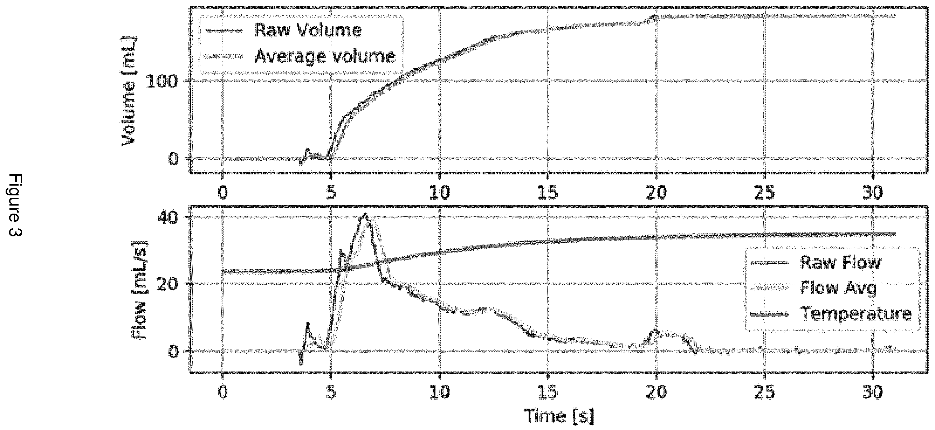

Figure 3 shows an example of results of urine flow analysis carried out with the described system. The upper panel shows a change in the volume of accumulated urine in time, while the bottom panel shows flow in ml/s and its variation in time. The measured temperature is also shown, which is due to presence of body fluid increased in the second part of the measurement. An example of the measured data shows the final urine volume as plateau at value 180 ml and return to value zero at the flow graph, while the maximal flow is shown as the highest slope on the volume graph and as the peak in the flow graph at value 40 ml/s.

## Claims

1. A device for measurement of urine flow, wherein the device is made of non-rigid resilient material and comprises:
- at least two containers, an inner (2) and an outer (1), wherein:
∘ the inner (2) and the outer (1) container are shaped as a truncated prism or pyramid having six sides, three narrower (B) and three wider (A) arranged in an alternating manner, and the inner container (2) is arranged to be installed inside the outer container (1), wherein the inner (2) and the outer (1) container may be separate pieces or may be joined in one piece having said shape,
∘ the outer container (1) has an elongated neck (1a) and a bottom (1b) for receiving the inner container (2), wherein the inner container (2) ends with an inner bottom (2b) arranged to collect urine, wherein the said inner bottom (2b) is from the outer side provided with electronics for operation of the device, thus said electronics being protected in a space (5) between both bottoms (1b, 2b),
∘ flex capacitive electrodes (4) glued on the wider sides (A) of the truncated prism or pyramid on the outer surface of the inner container (2) are arranged to detect the level of urine on each of the wider sides, wherein the flex capacitive electrodes are provided with a shield and covered by the outer container (1), thus protecting the flex capacitive electrodes (4) from environment,
- the bottom of the device is also provided with at least three condensers and capacitive bridges formed by the flex capacitive electrodes and pins connected to a printed circuit,
- at least one sensor for sensing movement and tilting of the device, preferably an accelerometer and a gyroscope arranged to sense possible movement and tilting, and
- preferably at least one temperature sensor (9) arranged to detect urine temperature, wherein the said temperature sensor (9) protrudes from the bottom (2b) into the interior of the device.

2. The device for measurement of urine flow according to claim 1, **characterized in that** the inner and the outer container (1, 2) are separate pieces installed in a manner to create a space (5) between said containers, said space being arranged to receive electronics, and the upper edges of the inner and the outer container are joined in any suitable way, preferably by gluing or thermal coupling.

3. The device for measurement of urine flow according to any of the preceding claims, **characterized in that** the inner (2) and the outer container (1) are made from any suitable resilient washable material, preferably from thermoplastics.

4. The device for measurement of urine flow according to any of the preceding claims, **characterized in that** it further comprises a separate holder (3) for reliable installation on a toilet bowl of any shape, wherein the holder (3) is preferably shaped as a flat element with a central part with an opening (3a) in the middle, said opening (3a) being arranged to receive the device, and wherein the central part of the holder (3) has three sides (3b, 3c, 3d) for installation of the toilet bowl.

5. The device for measurement of urine flow according to any of the preceding claims, **characterized in that** it additionally comprises a sound sensor or a microphone (8) arranged to detect urine dripping.

6. The device for measurement of urine flow according to any of the preceding claims, **characterized in that** it further comprises a spectrophotometric device (7), preferably a 12-channel sensor for spectrophotometry arranged to illuminate the urine at different wavelengths, which further measures and estimates content of different substances in urine.

7. The device for measurement of urine flow according to any of the preceding claims, **characterized in that** it is further equipped with additional sensors and indicators in the bottom of the device, such as indicator lights for feedback about device operation, preferably LED RGB light integrated on the printed circuit.

8. The device for measurement of urine flow according to any of the preceding claims, **characterized in that** it is further provided with a display arranged to show measurement values at the bottom of the device.

9. The device for measurement of urine flow according to any of the preceding claims, **characterized in that** the device is further provided with additional electronic components that enable autonomous operation of the device, namely:
- a memory for storing data from sensors and electrodes,
- a processor for processing said data,
- a suitable transmitter for transferring said data in any suitable way, preferably via Bluetooth,
- a power source and a suitable battery, which may be any battery, a rechargeable or a replaceable battery.

10. A system for diagnostics of micturition disorders comprising the device for measurement of urine flow according to any of the preceding claims and an online analytical application or a platform, respectively, and a mobile application installed on a mobile or a smart phone, tablet, computer or similar device, wherein the online and the mobile application are arranged to show measurement data.

11. A method of use of the system according to claim 10, said method comprising the following steps:
a) installation of the device according to any claim from 1 to 9 on a suitable flat surface or preferably installation of the device according to claim 4 on a toilet bowl via the holder with an opening arranged to receive the device,
b) collecting urine sample into the device prepared in step a), wherein the flex capacitive electrodes measure the urine level on each wall of the device where electrodes are installed,
c) preferably sensing sounds of urine drops using the sound sensor or a microphone (8) arranged to detect urine dripping and/or spectrophotometric analysis of collected urine using the spectrophotometric device (7), preferably a 12-channel sensor for spectrophotometry arranged to illuminate the urine at different wavelengths,
d) sensing tilt of the device or its movement with the sensor for sensing movement and tilting of the device, preferably an accelerometer and/or gyroscope,
e) sending data obtained from the electrodes and sensors in steps b), c) and/or d) in real time to the processor and the memory of the device, wherein the device gives an information about millilitres per second with a time resolution of at least one point per second, preferably 10 points per second,
f) processing of the data acquired in step e) in order to obtain at least one, preferably all of the following parameters:
- total time of urination,
- total volume,
- maximal urine flow, and
- time needed to reach the maximal urine flow
wherein the measurements are averaged and optionally adjusted with regards to the sensed urine temperature using the temperature sensor, or tilt of the device using the at least one sensor for sensing movement and tilting of the device, preferably an accelerometer and a gyroscope arranged to sense possible movement and tilting.

12. The method according to the preceding claim, **characterized in that** steps b), c) and d) are carried out simultaneously, sequentially, or two simultaneously and one later on.

## Patentansprüche

1. Vorrichtung zur Messung von Harnfluss, wobei die Vorrichtung aus einem nicht starren, elastischen Material besteht und umfasst:
- mindestens zwei Behälter, einen inneren (2) und einen äußeren (1), wobei:
∘ der innere (2) und der äußere (1) Behälter als abgestumpftes Prisma oder Pyramide mit sechs Seiten geformt sind, drei schmalere (B) und drei breitere (A), die abwechselnd angeordnet sind, und der innere Behälter (2) angeordnet ist, um innerhalb des äußeren Behälters (1) installiert werden zu können, wobei der innere (2) und der äußere (1) Behälter separate Teile sein können oder zu einem einzigen Teil mit dieser Form verbunden sein können,
∘ der äußere Behälter (1) einen länglichen Hals (1a) und einen Boden (1b) zum Aufnehmen des inneren Behälters (2) aufweist, wobei der innere Behälter (2) mit einem inneren Boden (2b) endet, der angeordnet ist, um Harn zu sammeln, wobei der innere Boden (2b) von der Außenseite mit Elektronik für den Betrieb der Vorrichtungversehen ist, sodass die Elektronik in einem Raum (5) zwischen den beiden Böden (1b, 2b) geschützt ist,
∘ flexible kapazitive Elektroden (4), die auf den breiteren Seiten (A) des abgestumpften Prismas oder der Pyramide auf die äußere Oberfläche des inneren Behälters (2) geklebt sind, die angeordnet sind, um den Harnpegel an jeder der breiteren Seiten zu detektieren, wobei die flexiblen kapazitiven Elektroden mit einer Abschirmung versehen und von dem äußeren Behälter (1) bedeckt sind, sodass die flexiblen Elektroden (4) vor Umwelteinflüssen geschützt sind,
- der Boden der Vorrichtung außerdem mit mindestens drei Kondensatoren und kapazitiven Brücken versehen ist, die durch die flexiblen kapazitiven Elektroden und mit einer Leiterplatte verbundene Kontaktstifte gebildet werden,
- mindestens einen Sensor zum Erfassen von Bewegung und Neigen der Vorrichtung, vorzugsweise einen Beschleunigungssensor und ein Gyroskop, die angeordnet sind, um mögliche Bewegungen und Neigungen zu erfassen, und
- vorzugsweise mindestens einen Temperatursensor (9), der angeordnet ist, um die Harntemperatur zu detektieren, wobei der Temperatursensor (9) aus dem Boden (2b) in das Innere der Vorrichtung hineinragt.

2. Vorrichtung zur Messung von Harnfluss nach Anspruch 1, **dadurch gekennzeichnet, dass** der innere und der äußere Behälter (1, 2) separate Teile sind, die in einer Weise installiert sind, dass ein Raum (5) zwischen den Behältern gebildet wird, wobei der Raum zum Aufnehmen von Elektronik ausgebildet ist, und dass die oberen Ränder des inneren und des äußeren Behälters auf beliebige geeignete Weise miteinander verbunden sind, vorzugsweise durch Kleben oder thermische Kopplung.

3. Vorrichtung zur Messung von Harnfluss nach einem der vorstehenden Ansprüche, **dadurch gekennzeichnet, dass** der innere (2) und der äußere Behälter (1) aus einem beliebigen geeigneten, elastischen und abwaschbaren Material bestehen, vorzugsweise aus Thermoplasten.

4. Vorrichtung zur Messung von Harnfluss nach einem der vorstehenden Ansprüche, **dadurch gekennzeichnet, dass** sie ferner eine separate Halterung (3) für eine zuverlässige Installation an einer Toilettenschüssel beliebiger Form umfasst, wobei die Halterung (3) vorzugsweise als flaches Element mit einem Mittelteil mit einer mittigen Öffnung (3a) geformt ist, wobei die Öffnung (3a) zum Aufnehmen der Vorrichtung angeordnet ist, und wobei der Mittelteil der Halterung (3) drei Seiten (3b, 3c, 3d) für die Installation der Toilettenschüssel aufweist.

5. Vorrichtung zur Messung von Harnfluss nach einem der vorstehenden Ansprüche, **dadurch gekennzeichnet, dass** sie zusätzlich einen Schallsensor oder ein Mikrofon (8) umfasst, der/das angeordnet ist, um das Tropfen von Harn zu detektieren.

6. Vorrichtung zur Messung von Harnfluss nach einem der vorstehenden Ansprüche, **dadurch gekennzeichnet, dass** sie ferner eine spektralphotometrische Vorrichtung (7) umfasst, vorzugsweise einen 12-Kanal-Sensor für Spektralphotometrie, der angeordnet ist, um den Harn mit unterschiedlichen Wellenlängen zu bestrahlen, der außerdem den Gehalt unterschiedlicher Substanzen im Harn misst und schätzt.

7. Vorrichtung zur Messung von Harnfluss nach einem der vorstehenden Ansprüche, **dadurch gekennzeichnet, dass** sie ferner mit zusätzlichen Sensoren und Indikatoren in dem Boden der Vorrichtung ausgestattet ist, wie Anzeigeleuchten für die Rückmeldung über den Betrieb der Vorrichtung, vorzugsweise mit auf der Leiterplatte integrierten RGB-LEDs.

8. Vorrichtung zur Messung von Harnfluss nach einem der vorstehenden Ansprüche, **dadurch gekennzeichnet, dass** sie ferner mit einer Anzeige versehen ist, die angeordnet ist, um die Messwerte an dem Boden der Vorrichtung anzuzeigen.

9. Vorrichtung zur Messung von Harnfluss nach einem der vorstehenden Ansprüche, **dadurch gekennzeichnet, dass** sie ferner mit zusätzlichen elektronischen Komponenten versehen ist, die den autonomen Betrieb der Vorrichtung ermöglichen, nämlich:
- einem Speicher zum Speichern von Daten von Sensoren und Elektroden,
- einem Prozessor zum Verarbeiten der Daten,
- einem geeigneten Sender zum Übertragen der Daten auf beliebige geeignete Weise, vorzugsweise über Bluetooth,
- einer Stromquelle und einer geeigneten Batterie, bei der es sich um eine beliebige Batterie, einen Akkumulator oder eine austauschbare Batterie handeln kann.

10. System zur Diagnose von Miktionsstörungen, das die Vorrichtung zur Messung von Harnfluss nach einem der vorstehenden Ansprüche bzw. eine Online-Analyseanwendung oder -Plattform und eine auf einem Mobiltelefon oder einem Smartphone, Tablet, Computer oder einem ähnlichen Gerät installierte mobile Anwendung umfasst, wobei die Online- und die mobile Anwendung dafür vorgesehen sind, Messdaten anzuzeigen.

11. Verfahren zur Verwendung des Systems nach Anspruch 10, wobei das Verfahren die folgenden Schritte umfasst:
a) Installation der Vorrichtung nach einem der Ansprüche 1 bis 9 auf einer geeigneten ebenen Oberfläche oder vorzugsweise Installation der Vorrichtung nach Anspruch 4 auf einer Toilettenschüssel über die Halterung mit einer Öffnung, die angeordnet ist, um die Vorrichtung aufzunehmen,
b) Sammeln der Harnprobe in der in Schritt a) vorbereiteten Vorrichtung, wobei die flexiblen kapazitiven Elektroden den Harnpegel auf jeder Wand der Vorrichtung messen, an der Elektroden angebracht sind,
c) vorzugsweise Erfassen von Geräuschen von Harntropfen unter Verwendung des Schallsensors oder eines Mikrofons (8), die angeordnet sind, um Harntropfen zu detektieren, und/oder spektralphotometrische Analyse von gesammeltem Harn unter Verwendung der spektralphotometrischen Vorrichtung (7), vorzugsweise ein 12-Kanal-Sensor für Spektralphotometrie, der angeordnet ist, um den Harn mit unterschiedlichen Wellenlängen zu bestrahlen,
d) Erfassen der Neigung der Vorrichtung oder ihrer Bewegung mit dem Sensor zum Erfassen von Bewegung und Neigung der Vorrichtung, vorzugsweise einem Beschleunigungsmesser und/oder Gyroskop,
e) Senden der in Schritten b), c) und/oder d) von den Elektroden und Sensoren erhaltenen Daten in Echtzeit an den Prozessor und den Speicher der Vorrichtung, wobei die Vorrichtung eine Information über Milliliter pro Sekunde mit einer Zeitauflösung von mindestens einem Messpunkt pro Sekunde, vorzugsweise 10 Messpunkten pro Sekunde, angibt,
f) Verarbeiten der in Schritt e) erfassten Daten, um mindestens einen, vorzugsweise alle der folgenden Parameter zu erhalten:
- Gesamtzeit des Harnflusses,
- Gesamtvolumen,
- maximaler Harnfluss, und
- benötigte Zeit, um den maximalen Harnfluss zu erreichen,
wobei die Messwerte gemittelt und optional unter Berücksichtigung der unter Verwendung des Temperatursensors erfassten Harntemperatur oder der Neigung der Vorrichtung unter Verwendung des mindestens einen Sensors zum Erfassen von Bewegung und Neigung der Vorrichtung, vorzugsweise eines Beschleunigungssensors und eines Gyroskops, die angeordnet sind, um mögliche Bewegungen und Neigungen zu erfassen, angepasst werden.

12. Verfahren nach dem vorstehenden Anspruch, **dadurch gekennzeichnet, dass** Schritte b), c) und d) gleichzeitig, nacheinander oder zwei gleichzeitig und einer später ausgeführt werden.

## Revendications

1. Dispositif de mesure de débit urinaire et système de diagnostic de troubles de la miction comprenant ledit dispositif, lequel est constitué d'un matériau élastique non-rigide et comprend :
- au moins deux récipients, l'un intérieur (2) et l'autre extérieur (1), où :
∘ les récipients intérieur (2) et extérieur (1) ont la forme d'un prisme ou d'une pyramide tronqué(e) à six faces, trois étroites (B) et trois larges (A) disposées en alternance, et le récipient intérieur (2) est conçu pour être installé à l'intérieur du récipient extérieur (1), les récipients intérieur (2) et extérieur (1) pouvant être des pièces séparées ou être assemblés en une pièce unique ayant ladite forme,
∘ le récipient extérieur (1) a un col allongé (1a) et un fond (1b) destiné à recevoir le récipient interne (2), le récipient interne (2) se terminant par un fond interne (2b) conçu pour recueillir de l'urine, où ledit fond interne (2b) est pourvu sur sa face externe d'un circuit électronique destiné à faire fonctionner le dispositif, ledit circuit électronique étant protégé dans un espace (5) entre les deux fonds (1b, 2b),
∘ des électrodes capacitives flexibles (4) collées sur les faces larges (A) du prisme ou de la pyramide tronqué(e) sur la surface extérieure du récipient intérieur (2) sont conçues pour détecter le niveau d'urine sur chacune des faces larges, les électrodes capacitives flexibles étant pourvues d'un blindage et recouvertes par le récipient extérieur (1), afin de protéger les électrodes capacitives flexibles (4) de l'environnement extérieur,
- en outre le fond du dispositif comprend au moins trois condensateurs et des ponts capacitifs formés par les électrodes capacitives flexibles et des broches connectées à un circuit imprimé,
- au moins un capteur destiné à détecter le mouvement et l'inclinaison du dispositif, de préférence un accéléromètre et un gyroscope conçus pour détecter des mouvements et une inclinaison éventuels, et
- de préférence au moins un capteur de température (9) conçu pour détecter la température de l'urine, ledit capteur de température (9) faisant saillie sur le fond (2b) et pénétrant à l'intérieur du dispositif.

2. Dispositif de mesure de débit urinaire selon la revendication 1, **caractérisé par le fait que** les récipients intérieur et extérieur (1, 2) sont des pièces séparées installées de façon à créer un espace (5) entre lesdits récipients, ledit espace étant conçu pour recevoir le circuit électronique, et les bords supérieurs des récipients intérieur et extérieur sont assemblés d'une façon appropriée quelconque, de préférence par collage ou thermosoudage.

3. Dispositif de mesure de débit urinaire selon l'une quelconque des revendications précédentes, **caractérisé par le fait que** les récipients intérieur (2) et extérieur (1) sont constitués de tout matériau élastique lavable approprié, de préférence un matériau thermoplastique.

4. Dispositif de mesure de débit urinaire selon l'une quelconque des revendications précédentes, **caractérisé par le fait qu'**il comprend également un support séparé (3) destiné à une installation fiable sur une cuvette de toilette de quelque forme que ce soit, où le support (3) a de préférence la forme d'un élément plat avec une partie centrale comportant une ouverture (3a) en son centre, ladite ouverture (3a) étant conçue pour recevoir le dispositif, et où la partie centrale du support (3) a trois côtés (3b, 3c, 3d) pour l'installation sur la cuvette des toilettes.

5. Dispositif de mesure de début urinaire selon l'une quelconque des revendications précédentes, **caractérisé par le fait qu'**il comprend en outre un capteur sonore ou un microphone (8) conçu pour détecter l'écoulement de l'urine.

6. Dispositif de mesure de débit urinaire selon l'une quelconque des revendications précédentes, **caractérisé par le fait qu'**il comprend également un dispositif spectrophotométrique (7), de préférence un capteur spectrophotométrique à 12 canaux, conçu pour éclairer l'urine à différentes longueurs d'onde, qui en outre mesure et évalue la teneur en diverses substances de l'urine.

7. Dispositif de mesure de débit urinaire selon l'une quelconque des revendications précédentes, **caractérisé par le fait qu'**il est également doté de capteurs supplémentaires et d'indicateurs au niveau du fond, tels que des témoins lumineux renseignant sur le fonctionnement du dispositif, de préférence une LED RGB intégrée au circuit imprimé.

8. Dispositif de mesure de débit urinaire selon l'une quelconque des revendications précédentes, **caractérisé par le fait qu'**il est aussi pourvu d'un afficheur conçu pour montrer les valeurs mesurées, au bas du dispositif.

9. Dispositif de mesure de débit urinaire selon l'une quelconque des revendications précédentes, **caractérisé par le fait qu'**il est également pourvu de composants électroniques supplémentaires qui permettent le fonctionnement autonome du dispositif, à savoir :
- une mémoire destinée à stocker les données provenant des capteurs et électrodes,
- un processeur destiné à traiter lesdites données,
- un module de transmission approprié, destiné au transfert desdites données, de préférence via Bluetooth,
- une source d'alimentation et une batterie appropriée, qui peut être tout type de batterie, une batterie rechargeable ou remplaçable.

10. Système de diagnostic de troubles de la miction comprenant le dispositif de mesure de débit urinaire selon l'une quelconque des revendications précédentes et une application d'analyse en ligne ou une plateforme, et une application mobile installée sur un téléphone portable ou un smartphone, une tablette, un ordinateur ou un dispositif similaire, où les applications en ligne et mobile sont conçues pour montrer les données mesurées.

11. Méthode d'utilisation du système selon la revendication 10, ladite méthode comprenant les étapes suivantes :
a) l'installation du dispositif selon l'une quelconque des revendications 1 à 9 sur une surface plate appropriée ou de préférence l'installation du dispositif selon la revendication 4 sur une cuvette de toilette par le biais du support doté d'une ouverture conçue pour recevoir le dispositif,
b) le recueil d'un échantillon d'urine dans le dispositif préparé à l'étape a), où les électrodes capacitives flexibles mesurent le niveau de l'urine sur chaque paroi du dispositif où les électrodes sont installées,
c) de préférence la détection des sons produits par l'écoulement de l'urine à l'aide du capteur sonore ou d'un microphone (8) conçu pour détecter l'écoulement de l'urine, et/ou l'analyse spectrophotométrique de l'urine recueillie à l'aide du dispositif spectrophotométrique (7), de préférence un capteur spectrophotométrique à 12 canaux conçu pour éclairer l'urine à différentes longueurs d'onde,
d) la détection de l'inclinaison du dispositif ou de ses mouvements à l'aide du capteur destiné à la détection des mouvements et de l'inclinaison du dispositif, de préférence un accéléromètre et/ou un gyroscope,
e) l'envoi des données procurées par les électrodes et capteurs aux étapes b), c) et/ou d) en temps réel au processeur et à la mémoire du dispositif, le dispositif donnant des informations concernant les millilitres par seconde avec une résolution temporelle d'au moins un point de mesure par seconde, de préférence 10 points de mesure par seconde,
f) le traitement des données acquises à l'étape e) afin d'obtenir au moins un, de préférence la totalité des paramètres suivants :
- la durée totale de miction,
- le volume total,
- le débit urinaire maximal, et
- le temps nécessaire pour atteindre le débit urinaire maximal
où l'on fait la moyenne des mesures et où elles sont facultativement ajustées en prenant en compte la température de l'urine détectée à l'aide du capteur de température, ou l'inclinaison du dispositif à l'aide d'au moins un capteur destiné à la détection des mouvements et de l'inclinaison du dispositif, de préférence un accéléromètre et un gyroscope conçus pour détecter les éventuels mouvements et inclinaison.

12. Méthode selon la revendication précédente, **caractérisée par le fait que** les étapes b), c) et d) sont réalisées simultanément, successivement, ou deux simultanément et une autre ultérieurement.
